# EUROPEAN PATENT APPLICATION

(11) **EP 2 786 773 A1**
(43) Date of publication of application: **08.10.2014**
(21) Application number: 12854367.5
(22) Date of filing: 28.11.2012
(51) Int. Cl.: A61M 5/00, A61B 5/00, A61M 5/142, A61M 5/145

(54) **FLUID-INJECTION SYSTEM FOR MEDICAL USE IN THE VASCULAR SYSTEM OF A PATIENT**

(30) Priority: 02.12.2011 ES 201131956 P
(71) Applicant: Costovici, Nicolas Anthony, 17480 Roses - Girona (ES)
(72) Inventor: Costovici, Nicolas Anthony, 17480 Roses - Girona (ES)
(74) Representative: Gallego Jiménez, José Fernando
(86) International application number: PCT/ES2012/000296
(87) International publication number: WO 2013/079743

(57) **Abstract**

The invention relates to a system which can be used for injecting liquids as well as gases, and includes at least: one pipe for inputting gas into the system, with a device controlling the pressure of the input gas; a gas-injection device provided with a means for adjusting a flow of gas to be supplied to a patient, at a predetermined pressure and with at least one flow sensor that determines the exact amount of gas supplied to said patient; a kit for administering gas to the patient; and at least one catheter or needle that can be connected to said administration kit and to the patient, with a drain device arrange between the administration kit and the injection device.

## Description

### Object of the invention

The present invention relates to a system for injecting fluids, preferably gases, for medical use in the vascular system of a patient. This system presents some features aimed to reliably control the amount of gas or fluid introduced into the patient and allowing the establishment of a delivery protocol extended in time, for example during the performance of a test or during a surgical procedure.

### Technical field of the invention

This invention is applicable in the medical-surgical field.

### Background of the invention

The systems currently available for injecting gases into the vascular system of patients use syringes. Syringes are filled with gas under a pressure which is variable. Based on the pressure in the syringe and the syringe volume it is possible to determine the gas volume injected. This method has several disadvantages:
- The preparation of the syringe is difficult.
- The volume injected into the patient is not a parameter that can be selected directly, because this volume must be calculated based on the pressure in the syringe and the syringe volume.
- A syringe, or two syringes if a dual-head gas injector is used, must be emptied. Therefore, there is no easy and immediate solution to perform examination again if something were not right.
- It is not possible to maintain a continuous injection for a long time.

For vascular or procedural radiology or to apply anaesthetic or the like, a continuous injection is needed.

In certain procedures of angiography and CT (computed tomography) imaging, it is usual to apply injections of iodine-based contrast media to the patient, which may cause allergic reactions and affect kidney and liver functions.

In other procedures, such as for the placement of a stent, several injections of iodine-based contrast media are needed.

One option to avoid the application of these injections of iodine-based contrast media is the use of CO2, which avoids the liver and kidney toxicity produced by multiple injections of iodine-based contrast media. However the delivery of CO2 through syringes prevents a continuous gas application to the patient and is complicated because of the difficulty of calculating the volume of gas applied mainly due to the variation of volume in accordance with the pressure.

### Description of the invention

The system of the invention presents some features aimed to allow the programming of complex supply protocols with one or more gases or fluids that will open new fields for anaesthetic medicine.

This invention provides the radiologist with a system that allows fluid to be prepared for injection to the patient for a new examination in a short time, of less than one minute, which is a significant advantage over existing gas injectors using syringes that are slow and complex to use.

Another objective of the invention is to provide security for both the patient and medical staff as it allows for very accurately determining the volume of gas injected into the patient.

The system makes it possible to incorporate means for heating the fluid or gas to be injected in order to provide greater comfort to the patient in those procedures in which the patient has no anaesthetic.

The system includes a three-function valve with a safety mechanical control, which ensures the possibility for the operator to control the machine and intervene in case of emergency.

For angiography and CT (computed tomography) imaging procedures, this system offers an alternative to the injection of iodine-base contrast media, by injecting CO2, which eliminates allergic reactions and avoid any impact on kidney function.

This solution is also inexpensive, as the use of CO2 is cheaper than the use of iodine-base contrast media.

For example, for the placement of a stent, several injections of iodine-based contrast media are needed. The use of this invention with CO2 is the best solution to avoid liver and kidney toxicity produced by multiple injections of iodine-based contrast media.

The use of gas rather than iodine-based contrast media is better for dilution.

Gases, and especially CO2, have less viscosity than iodine-based contrast media, they expand when flowing out and don't dilute with grouped haemorrhages. This may reveal abnormalities such as fistula, collateral vessels or arterial draining that were not possible to detect by means of liquid contrast.

The low viscosity allows the injection of CO2 by means of smaller application (delivery) devices and provides visualization of structures that cannot be seen with a liquid contrast medium.

Furthermore, CO2 can also be injected by means of smaller catheters and balloons without removing the guide wire. This enhancement boosts the efficiency and safety of the procedure.

The injection of gas in blood is currently used to replace some products such as enemas, contrast media or other drugs. CO2 is the most currently used gas due its neutrality and because its lack of side effects.

Therefore, another objective of the invention is to develop an injection system of fluids, and especially CO2, or any other medical gas in a safe and controlled manner.

The system of the invention comprises, at least:
- one pipe for inserting gas to the system,
- a device controlling the pressure of the gas from the gas input pipe, said controlling device being responsible for determining the pressure of the input gas to the device for injecting gas;
- a gas-injection device provided with means for adjusting a flow of gas to be supplied to a patient, at a predetermined pressure by the pressure controlling device; and at least one flow sensor to determine the exact amount of gas supplied to said patient,
- a kit for administering gas to the patient,
- at least one catheter or needle that can be connected to said supply kit and to the patient to be treated.

The pressure controlling device allows ensuring that the fluid or gas to be supplied passes to the injection device at a predetermined pressure, which is lower than the pressure at which the gas enters the input pipe. The adjusting of the pressure and thus of the supply of fluid or gas to the injection device at a constant and determined pressure allows to easily quantify the amount of product delivered to the patient through the injection device, unlike what occurs with the use of syringes whereby the gas is supplied at a variable pressure and therefore also at a variable volume.

The device controlling the pressure of the input gas to the system may further comprise means for heating the gas supplied to the gas injection device, and a device for adjusting the maximum flow of gas to be supplied to the injection device.

This injection device can have different configurations; for example it can be constituted by a peristaltic pump, which provides a certain volume of gas or fluid, as the input pressure is known and is determined by the above-mentioned pressure regulator. In an alternative embodiment, said injection device can be simply constituted by a valve which allows controlling the volume of fluid or gas passing at a predetermined and known pressure to the patient, based on the established prescription or program.

This system further comprises a control unit with a user interface for controlling and/or programming the operation of the injection system, both as regards the regulation of the input pressure and the volume of product that is injected into the patient.

This control unit allows to program the injection of gas or fluid for a certain amount of time, such as during the completion of an examination or procedure, and to always know the total amount of product that is being injected into the patient, this measure being provided by the flow sensor or sensors that are included in the injection device.

The system of the invention comprises a drain device arranged between the supply kit and the gas-injection device for draining the air contained in the supply kit and for filling said supply kit with the fluid or gas to be supplied before being injected into the patient.

This drain device comprises a three-position valve that is manually, semi-automatically or fully automatically operable, which comprises: a connecting pipe to the injection device, a connecting pipe to the supply kit and a connecting pipe to the outside; each of said connecting pipes having an opening and closing member for its selective interconnection in: a drain position, a preparing position and an injection position.

Said drain device comprises position sensors and is monitored by the control unit of the injection device for patient safety.

In a specific embodiment, the system of the invention comprises several gas and/or fluid input pipes that are connected to a supply kit by respective pressure controllers, respective injection devices and a common drain device. In this case, the system also comprises at least one additional injection device for pumping a physiological solution or a neutral solution and washing the drain device, the supply kit and the catheter if different gases or fluids to be supplied are to be injected separately, at different sequences and without them getting mixed.

In this case, the additional injection device is connected to the control unit, said control unit allowing the programmed activation of the additional injection device and the performance of a washing cycle between two independent injection cycles.

These and other features of the invention contained in the appended claims will become better understood in view of an exemplary embodiment of the invention.

### Description of figures

In order to complement the description that is being carried out and with the purpose of facilitating the understanding of the characteristics of the invention, the present description is accompanied by a set of drawings wherein, by way of a non-limiting example, the following has been represented:
- Figure 1 shows a diagram of an embodiment of the system for injecting medical fluids into the vascular system of a patient, according to the invention.
- Figure 2 shows an alternative embodiment of the previous figure, in which the system comprises a device for adjusting the maximum flow of gas or fluid that is supplied to the injection device, means for heating said gas or fluid, and a return pipe arranged between the injection device and the heating means for recirculation of the heated gas or fluid to be supplied.
- Figures 3a, 3b and 3c show respective embodiments of the valve of the drain device in the positions of: ready, injection and drain respectively.
- Figure 4 shows an embodiment of the system of the invention, which comprises several gas and/or fluid input pipes connected to a supply kit by respective pressure controlling devices, respective injection devices and a common drain device, and with an additional injection device for washing the drain device of the supply kit and of the catheter where different gases or fluids must be injected separately by the injection devices.

### Preferred embodiment of the invention

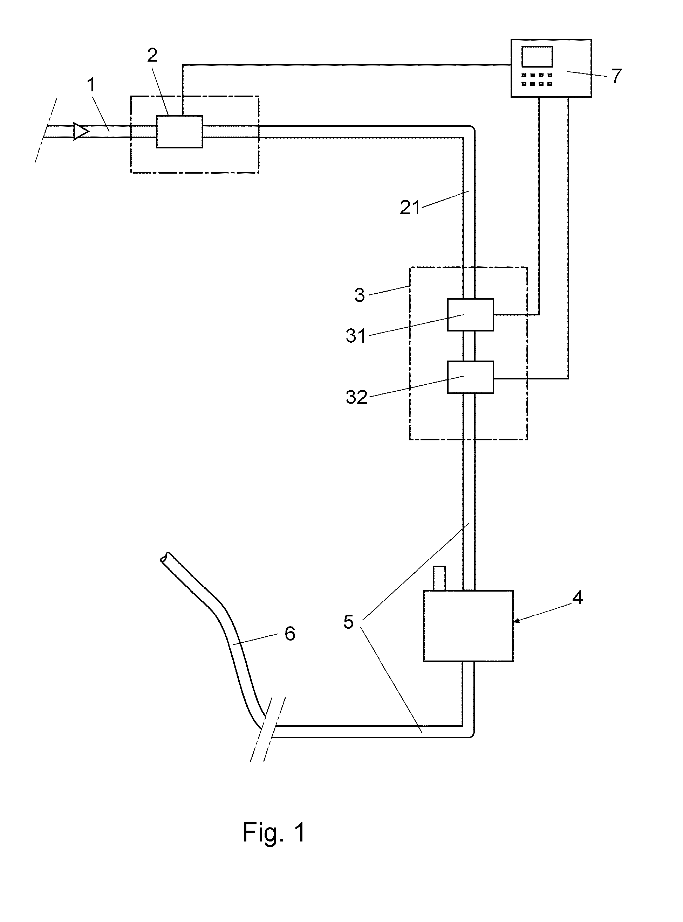

In the embodiment shown in Figure 1, the system for injection of fluids for medical use of the invention comprises one pipe (1) for inserting gas or fluid to be supplied to the system at a pressure that is higher than that at which said gas or fluid is to be supplied to the patient.

This input pipe (1) is connected to a device (2) controlling the pressure of the gas or fluid from the input pipe (1) and which determines the supply pressure of the gas to an injection device (3).

This injection device (3) is provided with means (31) for adjusting the flow of gas to be supplied to a patient, at the pressure that is determined by the device (2) for controlling the pressure, and at least one flow sensor (32) that is responsible for measuring the exact amount of product delivered to the patient.

In the example shown in Figure 1, the device (2) for controlling the pressure is connected to the injection device (3) by means of a pipe (21); the system further comprises a drain device (4), a supply kit (5) of a single use, and a catheter (6) or needle for the injection of the gas to the patient.

The system is controlled by a control unit (7) which allows, by means of a user interface, for the control and/or programming of the operation of the various system components.

The means (31) for adjusting the flow can be, for example, a peristaltic pump, which is a recommended option because this type of pump does not provide any flow when it stops and when the pressure of the gas supplied by the means for adjusting the flow at the inlet of the pump is known, the amount in volume of gas that is injected to the patient can be directly known by programming the pump speed.

However, it is also possible to use other types of pumps or devices such as a simple opening or closing valve.

In this embodiment, the means (31) for adjusting the flow are controlled by the control unit (7).

The gas injection device (3) can be suitable to control the exact amount of volume of gas injected into the patient.

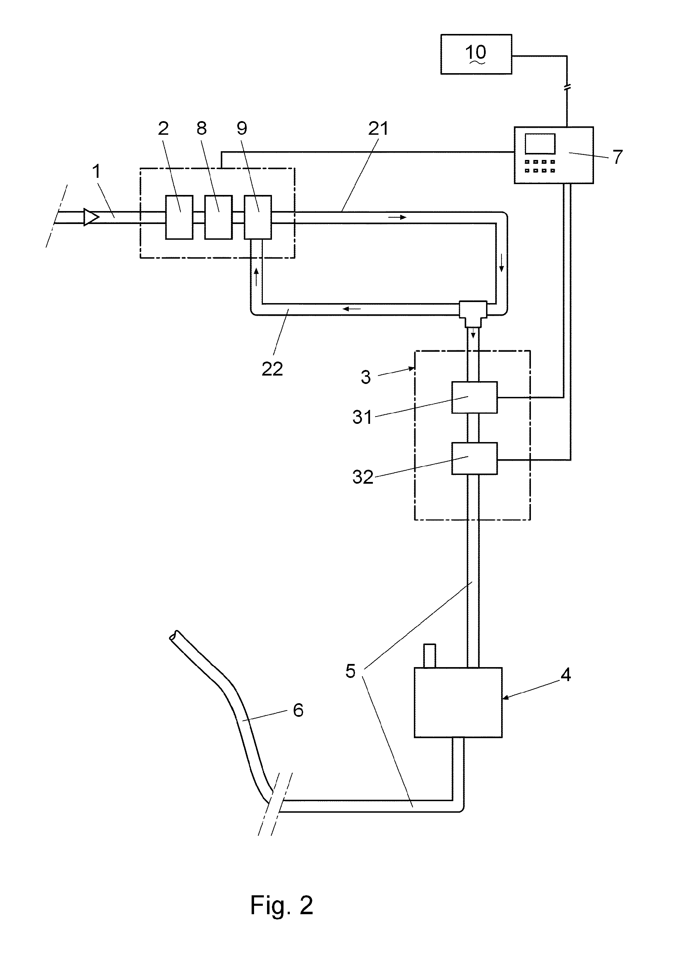

In the embodiment shown in Figure 2, the device (2) for controlling the pressure comprises an associated device (8) for controlling the maximum flow of gas or fluid to be supplied to the system and heating means (9) of gas or fluid to be supplied to the injection device (3). In this embodiment of the system, the device (2) for controlling the pressure is connected to the injection device (3) by means of a supply pipe (21) of gas or fluid and a return pipe (22) allowing to recirculate the gas or fluid through the heating means (9) and maintain it at all times at a desired temperature and pressure until being used by the injection device (3).

The drain device (4) is intended to perform the drain, i.e. extracting the air contained in the supply kit (5) and filling it with CO2 or other medical gas before injection into the patient.

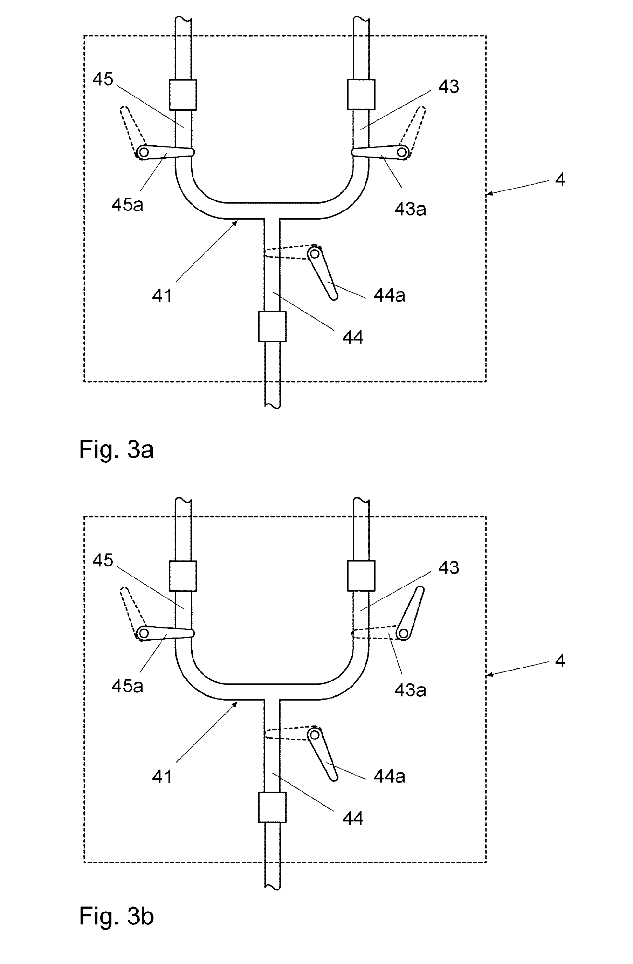

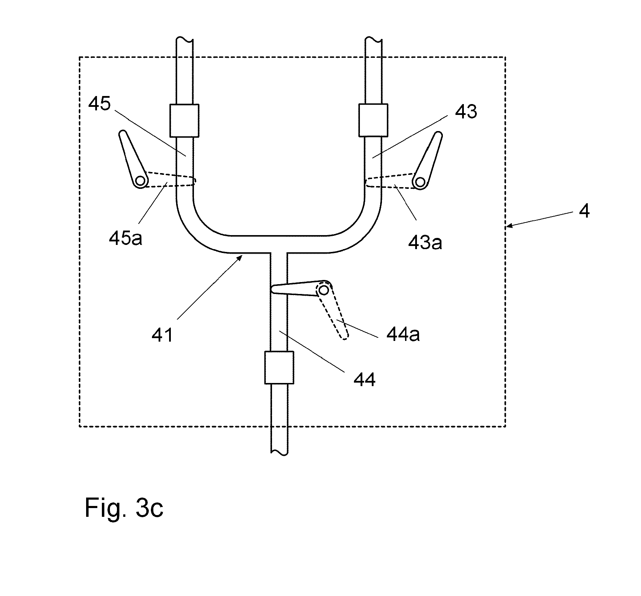

In the embodiment shown in Figures 3a, 3b, 3c, the drain device (4) comprises a mechanical drain valve (41) with three positions.

This valve (41) can be manual, semiautomatic or fully automatic; and it can also be controlled remotely.

In all the three cases, the valve (41) has position sensors (42) and is monitored by the control unit (7) of the injection system for patient safety. There is a permanent communication between the position sensors (42) of the valve and the control unit of the system.

The valve (41) comprises: a pipe (43) for connection to the injection device (3), a pipe (44) for connection to a patient catheter (6) by means of the supply kit (5), and a pipe (45) for connection to the outside.

Said pipes (43, 44, 45) have respective opening and closing members (43a, 44a, 45a) for their selective interconnection and for putting the valve (41) of the drain device in the following positions:
- a ready position shown in Figure 3a, - an injection position shown in Figure 3b and a drain position shown in Figure 3c.

The opening and closing members (43a, 44a, 45a) of the pipes are arranged: - in the drain position, in positions 1- 0 - 1 (open - closed - open); - in the ready position, in positions 0 - 1 - 0 (closed-open-closed) and; - in the injection position to the patient, in positions 1 - 1 - 0 (open-open-closed).

In manual operation, the operator manually selects the three positions of the valve (41) and the control unit (7) of the injection system asks the operator to select the mode in the user interface. In this manual operation the following steps are taken:
Step 1. - Connecting the supply kit (5) to the catheter (6) and connecting the supply kit (5) to the three-way valve (41) of the drain device. The closing member (44a) is kept closed while checking that the catheter is filled with blood.
Step 2. - Selecting the drain mode. It must be verified that the catheter valve is closed. Selecting "drain" when the gas-injection device (3) is ready.
Step 3. - Positioning the valve (41) in the "ready" position.

When the control unit (7) of the injection system detects from the position sensor (42) that the valve (41) of three functions is in the "ready" position the injection device (3) stops. The tube is filled with medical gas.

Step 4. - Positioning the valve (41) in the injection mode when the device is ready to inject. Then the injection device (3) discharges the selected volume of gas. Waiting for instruction to turn the valve into the "ready" position.

Step 5. - The control unit (7) of the injection system requests the operator to put the valve (41) into the "ready" position. The injection is complete.

The valve (41) may be motorized to allow a semi-automatic or fully automatic operation.

In a semi-automatic operation of the valve (41) the operator must be near the patient with the three-way valve in his/her hands, this operation being performed by taking the following steps:
Step 1. (Same as above). Connecting the supply set (5) to the catheter (6) and connecting the supply kit (5) to the three-way valve (41) of the drain device (4).

The valve (41) of the catheter is kept closed while checking that the catheter is filled with blood.

Step 2. Positioning the valve (41) in the "drain" mode when the device is ready.

When the control unit (7) of the injection system detects the drain position of the valve, the drain is carried out automatically and the control unit (7) automatically rotates the valve of three functions into the "ready" position by a motorized actuator (not shown).

Step 3. Not required for the semi-automatic mode.

Step 4. Putting the valve (41) in the injection position when the device is ready to inject. It automatically starts the injection, and, when the injection is complete, the control unit (7) automatically puts the valve (41) in the "ready" position.

This mode has a motorized valve that is controlled by the control unit of the injection system.

The operator can move the positions of the valve.

In the invention, a fully automatic operation of the valve (41) of the drain device is contemplated, particularly for a remote work in those areas that are exposed to radiation.

In this mode, the operator can control the positions of the valve (41) directly from the user interface of the control unit (7) of the system.

This operation can be performed directly from the control unit (7) or from a remote control by taking the following steps:
Step 1: Same as the manual valve of three functions.
Step 2: Pressing "drain" in the user interface of the control unit (7) of the injection system when it is ready for the drain process while checking that the catheter (6) is filled with blood. Maintaining the pipe of connection to the catheter closed. The drain is carried out automatically.
Step 3: Pressing "injection" in the user interface of the control unit of the injection system when it is ready for the injection process.

The control unit (7) processes for the injection and alert when the injection is performed.

The control unit (7) allows the control of the system in fully automatic mode with CT (computed tomography), MRI or any other external medical device.

This operation controlled by an external medical device is the same as the fully automatic mode discussed above, except that the user interface of the external medical device sends injection commands to the control unit (7) of the injection system. The control unit is not controlled by users but by an external medical device (10), shown schematically in Figure 2.

### - Specific case of CO2 injection

When an unlimited amount of CO2 is injected for an unlimited period, it is possible to program several protocols in the control unit (7) of the injection system that can be extended in time.

It may be suitable to have more time to observe the vascular system with radiology or computed tomography. This can be very useful when CO2 is used as contrast medium and when the radiologist is performing an interventional radiology (IR) or vascular interventional radiology (VIR).

During the (IR) or (VIR), it may be of interest to maintain a level of CO2 in the blood to give the radiologist the contrast required throughout the procedure.

To avoid saturation of CO2 that can cause disease, the system of the invention can be connected by means of the control unit to a single or multiple CO2 monitor system (capnography or other systems).

When the control unit detects a hazardous CO2 saturation, the injection is stopped or the injection flow is reduced to allow the patient's body to eliminate the CO2 and revert to a reasonable level.

In other medical applications, the gas may be replaced with a liquid for an application requiring a continuous injection. An application is anaesthetic, but other applications are possible. In this case, the injection device pumps into a reservoir of medical fluid, instead of receiving the gas.

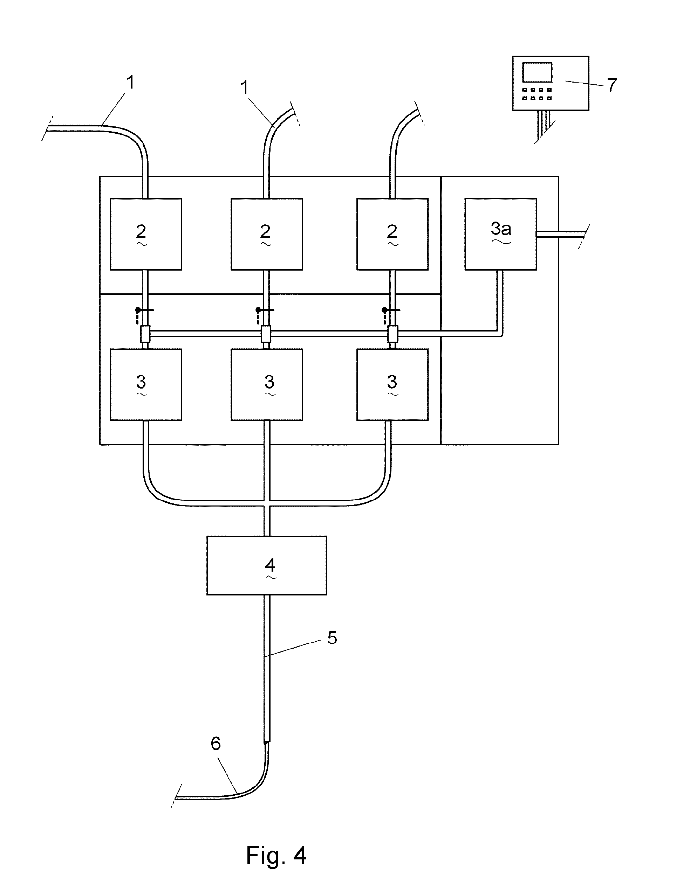

In the embodiment shown in Figure 4, the system comprises several input pipes (1) for gases and/or fluids connected to a supply kit (5) by means of respective devices (2) controlling the pressure, respective injection devices (3) and a common drain device (4), all being analogous to those described above. This system comprises at least one additional injection device (3a) for pumping a physiological solution or a neutral solution and washing the drain device (4), the supply kit (5) and the catheter (6) if different gases or fluids are to be injected separately through the injection devices, in different sequences, and without being mixed.

This additional injection device (3a) is connected to the control unit (7) as well as to the other members of the system; said control unit (10) allowing to program the activation of the additional injection device (3a) and carrying out a washing cycle from two independent injection cycles.

The system of the invention also allows the use of supply kits ready for use, such as vacuum supply kits or supply kits preloaded with CO2 or other medical gases. In these cases, the use of the drain device is required.

Once the nature of the invention as well as an example of preferred embodiment have been sufficiently described, it is stated for all pertinent purposes that the materials, form, size and arrangement of the members described are susceptible to changes, provided these do not involve an alteration of the essential characteristics of the invention that are claimed subsequently.

## Claims

1. System for the injection of medical fluids into the vascular system of a patient, which fluids may be liquids or gases for medical use, **characterized in that** it comprises at least:
- a pipe for inserting gas into the system,
- a device controlling the pressure of the gas from the gas insertion pipe, said controlling device being responsible for determining the pressure of the gas entering the system for injecting gas;
- a gas-injection device provided with means for adjusting a flow of gas to be supplied to a patient, at a predetermined pressure by the pressure controlling device; and at least one flow sensor determining the exact amount of gas supplied to said patient,
- a kit for supplying gas to the patient,
- at least one catheter or needle that can be connected to said supply kit and to the patient to be treated.

2. System according to claim 1, **characterized in that** it comprises a control unit with a user interface for controlling and/or programming the operation of the injection device.

3. System according to any one of the preceding claims, **characterized in that** it comprises a drain device arranged between the supply kit and the gas-injection device for draining the air contained in the supply kit and for filling said supply kit with CO2 or other gas before the injection of gas into the patient.

4. System according to claim 3, **characterized in that** the drain device comprises a three-position valve that is manually, semi-automatically or fully automatically operable comprising: an injection device connecting pipe, a supply kit connecting pipe and an outside connecting pipe, each of said connecting pipes being provided with an opening and closing member for being selectively interconnected in: a drain position, a ready position and an injection position.

5. System according to any one of claim 4, **characterized in that** the opening and closing members of the injection device, the supply kit and the outside connecting pipes are put: - in the drain position, in positions 1- 0 - 1 (open - closed - open); - in the ready position, in positions 0- 1 - 0 (closed-open-closed) and; - in the patient injection position, in positions 1 - 1 - 0 (open-open-closed).

6. System according to any one of claims 3 to 5, **characterized in that** the valve of the drain device comprises position sensors and is monitored by the control unit of the injection device for patient safety.

7. System according to any of the preceding claims, **characterized in that** the device controlling the pressure of the gas entering the system further comprises a device for adjusting the maximum flow of gas supply.

8. System according to any of the preceding claims, **characterized in that** the device controlling the pressure of the gas entering the system further comprises means for heating the gas supplied to the gas injection device.

9. System according to any of the preceding claims, **characterized in that** it comprises several pipes for inserting gases and/or fluids connected to a supply kit by means of respective pressure controllers, respective injection devices and a common drain device, and **in that** it comprises at least one additional injection device for pumping a physiological solution or a neutral solution and washing the drain device, the supply kit and the catheter in case the different gases or fluids are to be injected separately, in different sequences, and without being mixed.

10. System according to claim 9, **characterized in that** the additional injection device is connected to the control unit, said control unit allowing programming activation of the additional injection device and performing a wash cycle between two independent injection cycles.
